# EUROPEAN PATENT SPECIFICATION

(11) **EP 1 018 958 B1**
(45) Date of publication and mention of the grant of the patent: **09.02.2005**
(21) Application number: 98923877.9
(22) Date of filing: 02.06.1998
(51) Int. Cl.: A61B 18/12, A61B 18/14

(54) **ELECTRIC FIELD CONCENTRATED ELECTROSURGICAL ELECTRODE**
ELEKTOCHIRURGISCHE ELEKTRODE EIN ELEKTRISCHES FELD KONZENTRIEREND
ELECTRODE ELECTROCHIRURGICALE A CHAMP ELECTRIQUE CONCENTRE

(30) Priority: 03.10.1997 US 943551; 03.12.1997 US 984716
(43) Date of publication of application: 19.07.2000
(73) Proprietor: MEGADYNE MEDICAL PRODUCTS, INC., Draper, UT 84020 (US)
(72) Inventor: GREEP, Darcy, W., South Jordan, UT 84095 (US)
(74) Representative: Stuttard, Garry Philip
(86) International application number: PCT/US1998/011137
(87) International publication number: WO 1999/017670

(56) References cited:
- WO-A-97/11649
- US-A- 5 380 320
- US-A- 5 540 684
- US-A- 5 697 926
- US-A- 5 702 387

## Description

This invention relates to electrosurgery and more particularly to electrosurgical electrodes (eg probes, blades, forceps and the like) for use in performing electrosurgery. As known to those skilled in the art, modem surgical techniques typically employ radio frequency (RF) cautery to cut tissue and coagulate the same to stop bleeding encountered in performing surgical procedures. For historical perspective and details of such techniques, reference is made to US-A-4936842.

As known to those skilled in the medical arts, electrosurgery is widely used and offers many advantages including that of the use of a single surgical tool for both cutting and coagulation. A variety of proposals have heretofore been embodied in existing electrosurgical implements. Examples of such proposals include those set forth in US-A-4534347 (Taylor), US-A-4674498 (Stasz) and US-A-4785807 (Marsden Blanch).

The Taylor patent discloses an implement having a sharpened exposed edge (eg knife-blade like geometry) which is employed to perform conventional mechanical cutting of tissue whilst the blade is configured to act as a microwave radiator to transfer microwave energy by radiation into adjacent tissue to effect desired cauterization.

The Stasz patent sets forth several embodiments which disclose partly coated, partly exposed blades adapted for three modes of operation. These three modes are said to be: (1) a standard surgical cutting blade with a sharp edge when no electrical power is applied to it; (2) an electrocautery blade when a high voltage is applied between conductive surfaces of the blades sufficient to create a discharge arc therebetween for cutting and cauterizing of tissue; and (3) a low voltage cautery tool where I²R losses create heat to cauterize tissue.

The Blanch patent discloses an unsharpened blade which has been entirely coated with an insulating layer so that cutting is performed by electrical energy capacitively transferred through the insulating layer to the tissue which is to be cut rather than by conventional mechanical action. In such electrosurgergy, "cutting" is accomplished when energy transfer is sufficient to cause water in tissue cells to boil, thus rupturing the cell membranes by internal rather than external forces. Relatively high energy levels have been required to effect such electrosurgical cutting.

WO-A-97/11649 discloses an electrosurgical electrode member comprising an electrode with an insulating coating. Document US-A-5 380 320 discloses an electrosurgical electrode member for performing operative procedures on a patient comprising a conducting electrode having a main body adapted for communicating radio-frequency electrical energy to patient tissue for performing operative procedures thereupon, and an insulating coating covering said main body, said insulating coating being a parylene coating.

While the Blanch proposals have constituted an important advance in the art and have found widespread acceptance in the field of electrosurgery, there has been a continuing need for further improvement in electrosurgery to effect in a relatively simple geometric configuration, a reduction in thermal necrosis thereby decreasing postoperative complication, reducing eschar production, reducing incidence of heat damage to tissue away from the cutting site, and increasing the speed of cutting.

### BRIEF SUMMARY OF THE INVENTION

The present invention provides electrosurgical implements having a marked improvement in performance over the proposals heretofore made by achieving an important concentration of electrosurgical energy to permit more rapid and effective cutting at lower RF energy levels. In one embodiment, the electrosurgical implement includes a geometrical surface to be used to effect electrosurgical cutting. The geometrical surface is shaped to concentrate energy transfer. The shaped surface can include an edge or point of an electrically conducting interior part of the implement. The interior part of the implement is subsequently completely coated with insulation. As a result of the insulative coating, the shaped surface no longer presents a particularly sharp exterior geometrical surface for mechanical contact with patient tissue. Nevertheless, because of a concentration of electric field and energy transfer (as hereinafter described), the shaped surface provides a marked improvement in charge concentration and tissue severance to permit utilization of lower energy levels and results in reduced thermal necrosis, more rapid cutting, and reduced eschar production.

In alternative embodiments, electrosurgical implements of the present invention are characterized by geometrical configurations that include non-stick insulation on the outer surface of most of the electrosurgical implement. The electrosurgical implements also include an exposed, uncoated minute sharpened cutting or probing surface at which electrosurgical energy is concentrated for enhanced cutting (and, in certain cases, coagulating) characteristics. For probes and blades, the electrosurgical implement is specially adapted for use in either a cuffing or coagulation mode. In the cutting mode, the implement is positioned in a conventional cutting position in which the exposed blade or point is in contact with tissue that is to be cut. The presence of insulation over the blade other than at the point of cutting, together with the sharpened character of the exposed blade, concentrates the electrical energy at the point or line of contact. While there may be some incidental capacitive coupling to adjacent tissue, it is insufficient to produce noticeable effects.

When it is desired to provide coagulation, the relatively minute exposed cutting/probing surface is removed from tissue contact and one of the coated surfaces is disposed in contact with the tissue which is to be coagulated. This transition results in switching the mode of energy transfer from that of essentially ohmic conduction (in the cutting mode) to capacitive coupling energy transfer in the coagulation mode, and thus facilitating both cutting and coagulation at reduced power levels. The principles hereof may not only be applicable to blades, points and forceps, but also to modified ball electrodes, L-hooks, L-wires, J-hooks and similar constructions.

In another embodiment of the present invention, such as bi-polar forceps, it has been found that the principles of energy concentration may be advantageously employed to facilitate coagulation. There, since the forceps are not normally used for cutting, the energy concentration is embodied in a number of parallel edges that may be either partly exposed or entirely coated with non-stick coating depending upon the particular circumstances of use.

### BRIEF DESCRIPTION OF THE DRAWINGS

Figure 1 is a perspective view illustrating an implement representative of the prior art;
Figure 1A is a sectional view taken along section lines 1A- 1A of Figure 1;
Figure 2 is a perspective view of an implement embodying principles according to the present invention;
Figure 2A is a sectional view taken along the section lines 2A-2A of Figure 2 and depicting a partly sharpened working surface;
Figure 2B is a drawing similar to that of Figure 2A except for the working surface of the implement which is depicted as a knife edge;
Figure 3 is a view illustrating a typical electric field existing between a rounded surface implement and a working return electrode;
Figure 4 is a view illustrating modified electric field concentration associated with a sharply pointed geometry;
Figure 5 is a simplified view illustrating a typical concentration of electric field projected from the partly sharpened edge of Figure 2A;
Figure 6 is a view illustrating an alternative embodiment of the invention in which a minute region of a sharpened blade projects a predetermined distance outwardly from beneath a mostly coated blade to expose the edge;
Figure 7 is a view of a portion of the grasping surface of an electrosurgical forceps according to the prior art;
Figure 8 is a schematically depicted enlarged view of the grasping surface of the prior art forceps of Figure 7;
Figure 9 is a perspective view depicting a portion of the grasping surface of electrosurgical forceps according to the present invention;
Figure 10 is a schematically depicted enlarged view of the grasping surface of the forceps of Figure 9; and
Figure 11 is a cross section illustrating a further variation on the structure of Figures 6, 9 and 10 in which one extremity of a blade is coated, the opposite extremity includes an exposed Operative Surface as defined herein, and in which the sides of the blade include a series of abrupt geometrical anomalies to provide relatively sharp surface changes thereby to enhance energy concentration.

### DESCRIPTION OF A PREFERRED EMBODIMENT

As employed in this specification and the appended claims, the following terms shall have the meanings as now defined.

"Operative Surface" means an exposed surface of the energy-conducting member extending outwardly from beneath covering insulation by a distance of 0.2 mm or less.

"Non-stick Material" means any of a group of conventional and well-known materials used in this art as coatings to eliminate or reduce adherence of tissue, blood and the like to electrosurgical blades. Examples are coatings of diamond material or fluorinated hydrocarbons (PTFE), an example of the latter being that which is commercially available under the trade name TEFLON.

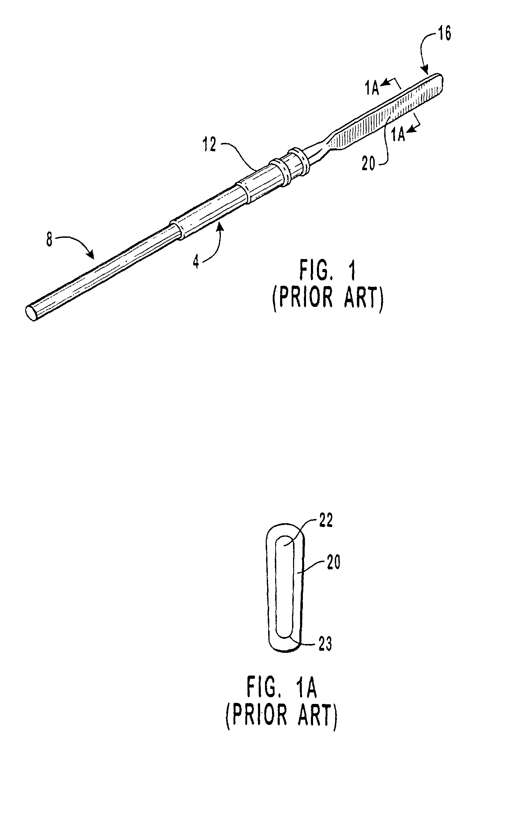

Turning to the drawings, and more particularly Figures 1 and 1 A thereof, depicted is an implement representative of the prior art as set forth in the aforementioned Blanch U.S. Patent No. 4,785,807. Specifically, the drawings depict an electrosurgical knife, generally shown at 4, having a proximal end 8 fitted with a sleeve fitting 12. Sleeve fitting 12 is positioned around the knife shank to provide protection and to facilitate holding of knife 4 by a conventional holder (not shown). Knife 4 also includes a distal end 16 formed with an unsharpened cutting surface 23 as shown. A coating 20 of non-stick material covers the surface area of the cutting blade 22 and serves to eliminate or reduce the clinging of charred tissue to the blade.

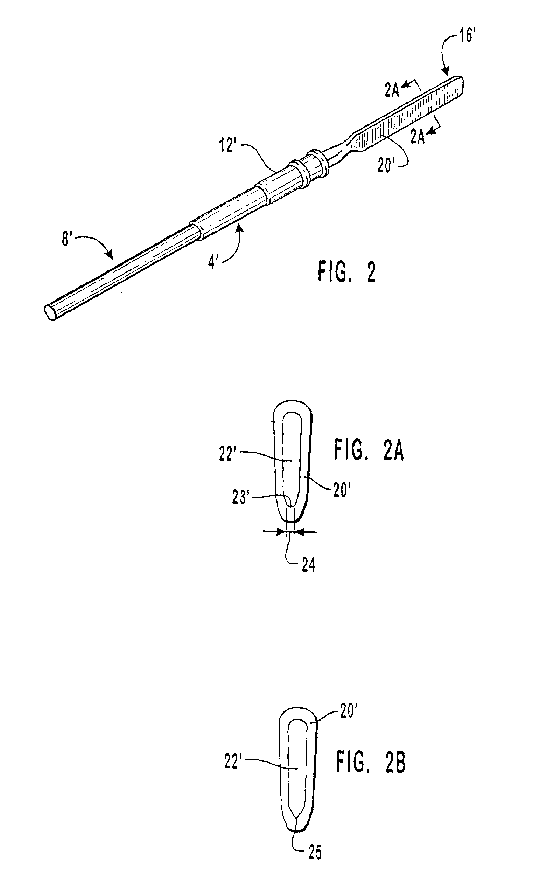

Figure 2 depicts an implement which embodies the principles underlying the present invention. Depicted in Figure 2 is an instrument appearing similar to that of Figure 1. Thus, in Figure 2 there is seen an electrosurgical knife, generally shown at 4', having a proximal end 8' fitted with a sleeve fitting 12'positioned around the knife shank. Sleeve fitting 12' provides protection and facilitates holding of knife 4' by a conventional electrosurgical holder (not shown). Knife 4' also includes a distal end 16' which is formed with a special geometrical shape as described in connection with Figures 2A, 4 and 5. A coating 20 of Non-stick Material covers the surface area of the cutting blade and serves to eliminate or reduce the clinging of charred tissue to the blade. In sharp contrast with the embodiment of Figure 1, however, the embodiment illustrated in Figure 2 features a cross sectional geometry which includes an edge that is at least partly sharpened as shown in Figure 2A.

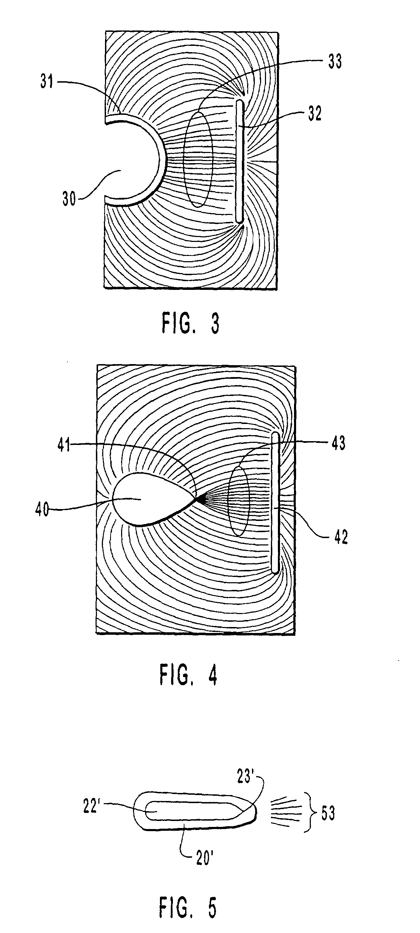

As mentioned above, Figure 2A is a sectional view taken along the section lines 2A-2A of Figure 2. Depicted therein is an electrically conductive main body 22' which may be of any suitable material such as, preferably, surgical grade stainless steel. Body 22' has been at least partly sharpened at its lower extremity to a knife edge or point 23'. As described in connection with Figures 3 and 4, sharpened portion 23' of body 22' concentrates or focuses the electric field created when electrical potential is applied to body 22'. In turn, this increases the concentration of transferred electrical energy and correspondingly improves the efficiency with which the implement achieves a cutting action, *e.g.*, severs tissue. Before leaving Figure 2A, it should be understood that while the preferred geometry embodies a fully sharpened edge (or point), such as that depicted in Figure 2B, the efficacious characteristics flowing from the invention begin to be significantly observed when the dimension 24 (*i*.*e*., working edge width) is at or less than 0.2 mm. This presents a working edge width of 0.2 mm or less. Such efficacious characteristics further improve as the dimension 24 is reduced to a knife edge.

Depicted in Figure 2B is a configuration similar to that of Figure 2A except that in Figure 2B there is depicted a fully sharpened blade having a knife edge 25. The physical principles underlying the foregoing marked improvement can be understood from reference to Figures 3 and 4. Figure 3 is a diagram illustrating electric field pattern lines for an electric field existing between a conductor or electrode 30 having an annular, or curved, exterior surface 31 and a counter electrode 32. Although electrode 30 is shown as being hollow, the electric field pattern shown is essentially the same if the electrode were solid. It will now be seen that the density of the electric field lines within ellipse 33 is nearly uniform and thus the electric field does not vary substantially within that region.

In Figure 4, however, it is noted that if the geometry of electrode 40 is made to include a pointed region as represented by point or edge 41. The corresponding electric field becomes much more concentrated at point or edge 41 as represented by the much greater line density of electric field lines (within the ellipse 43) between the electrode 40 and counter electrode 42. Thus, on an irregularly shaped conductor, charge tends to accumulate at locations where the curvature of the surface is greatest, that is, at sharp points or edges. By sharpening the blade edge in accordance with the present invention, the charge is concentrated along a much smaller surface area or region, thus focusing the electric field lines into a tighter arrangement. In turn, this reduces extraneous charge loss in tissue which is not in close proximity to the point or sharpened edge. The cutting edge of the electrode need not be sharply pointed. The cutting edge need only be shaped (sharpened) to concentrate energy transfer to the degree desired for optimum cutting.

By way of illustration, the conventional electrode of Figure 1 has an edge 23 thickness of about 0.33 mm and in the cutting mode may utilize a power setting nearing 40 watts. When sharpened to an edge 23' thickness of about 0.00735mm, a "sharpness" below that required of a mechanical scalpel blade, the electrode of Figure 2 can quickly cut through tissue at less than 20 watts; a power setting of 50% less than that required for the electrode of Figure 1. Moreover, the blade of Figure 2 cuts more rapidly with less resistance, less eschar production, less thermal necrosis, and improved operator control.

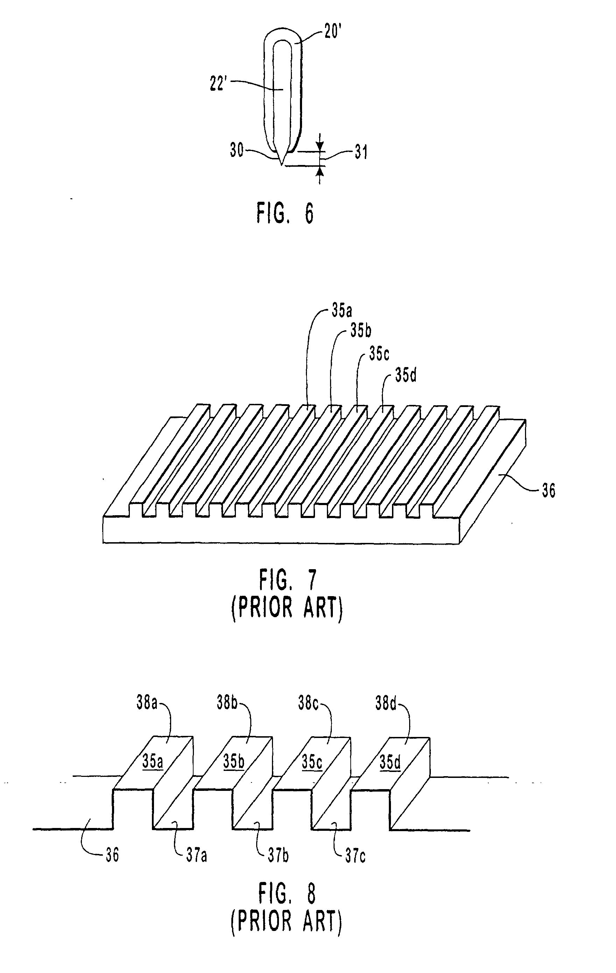

The foregoing principles are illustrated in Figure 5. As noted above, Figure 5 is a simplified view illustrating a typical concentration of electric field projected from the sharpened edge of Figure 2A. To facilitate clarity and simplicity of presentation, only lines 53 representing the electric field in the direction of the sharpened point or edge 23 are shown.

It is observed that the electrode of Figure 5 is the electrode illustrated earlier in Figure 2A. Thus, there is shown an electrically conductive main body 22' with an at least partly sharpened edge or point 23' completely coated with insulating coating 20'. When electrosurgical potential is applied to body 22' in the presence of tissue for which severance is desired, the density of energy transfer is concentrated at the apex 23' as represented by the longer rays within the bundle of rays 53. Thus, in the illustrated example, energy is concentrated along the principal axis of the main body extending from edge 23'.

The insulating coating 20' may be any of the known several Non-stick Materials (as defined above) that have been found attractive for use in electrosurgery and applied by any of the known techniques. However, in accordance with the one preferred embodiment hereof, such material is a fluorinated hydrocarbon (PTFE), an example of which is commercially available under the trade name TEFLON.

The thickness of the Non-stick Material of the embodiments of Figures 2A and 2B is that sufficient to ensure transmission of radio frequency electrical energy from the coated main body 22' to the tissue of the patient essentially exclusively by capacitive coupling, ordinarily less than 0.0254mm (1 mil). The precise optimum thickness will vary depending upon the material used and can be readily determined by routine experimentation. It is evident that this coating mechanically "dulls" any sharp electrode edge. As previously noted, however, cutting by electrosurgery does not necessarily require sharp surgical edges for mechanically severing tissue. Rather, the cutting is effected by utilizing sufficient energy to cause water in the tissue cells to boil and rupture the cell membranes.

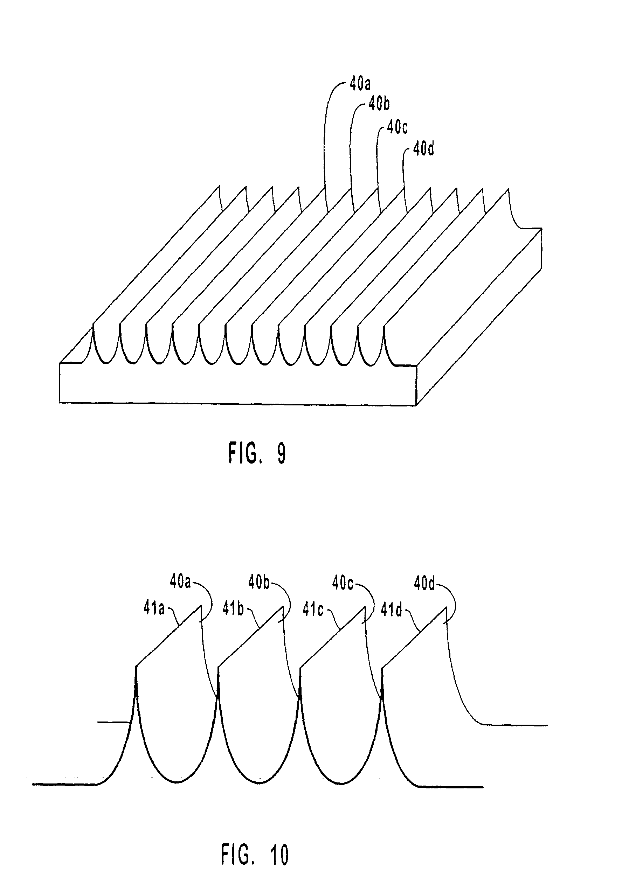

The energy concentration principles according to the present invention may additionally be effective when employed with an implement such as the embodiment of Figure 6. As depicted in Figure 6, electrically conducting main body 22' includes an exposed Operative Surface 30, as defined above, extending outwardly from beneath covering insulation 20'. Operative Surface 30 projects by a distance 31 of approximately 0.2 mm or less. It has been found that 0.2 mm marks the approximate limit of extension in typical surgical blades in order to achieve optimum energy concentration while preserving the insulation character of the remainder of the blade to facilitate coagulation. When in use, the energy concentrated at the exposed sharpened blade is particularly effective in achieving tissue severance. When it is desired to employ the blade in a coagulation mode, the exposed surface 30 is withdrawn from contact with tissue and the insulated side of the blade is disposed in contact with the area at which coagulation is desired. Thus, in the embodiment of Figure 6, tissue severance is achieved principally by ohmic conduction through concentration of energy at the sharpened surface, while coagulation is achieved principally through capacitive transfer of energy across the insulation 20'.

As mentioned above, the energy concentration principles according to the invention are applicable to other medical instruments such as forceps. Figures 7 and 8 illustrate typical bi-polar electrical forceps surfaces according to the prior art. Depicted in Figure 7 is a view of a portion of the grasping surface of a typical electrosurgical forceps according to the prior art. As will be observed, the prior art grasping surface has squared top ridges 35a, b, c and d (illustrated in enlarged form in Figure 8) projecting upwardly from principal surface 36. The square tops of these ridges are separated by generally rectangular valleys 37a, 37b and 37c. Thus, there are relatively broad area working surfaces 38a, 38b, 38c and 38d which result in correspondingly large surface contact areas with tissue when the forceps are in use.

Figures 9 and 10 are views generally similar to those of Figures 7 and 8 but depicting a portion of a grasping surface of electrosurgical forceps according to the present invention. As depicted in Figures 9 and 10, upwardly pointing projections 40a, 40b, 40c and 40d are sharpened to edges 41a, 41b, 41c and 41d and are operative to concentrate energy according to the principles described above in connection with Figures 2A, 2B and 6. Thus, such knife edges may be: slightly dulled and entirely coated with insulation as exemplified by Figure 2A; highly sharpened and entirely coated with insulation as exemplified by Figure 2B, or partly uncoated to present exposed operative surfaces as exemplified by Figure 6. In any event, the highly concentrated areas provided for engagement with patient tissue result in a high concentration of electrical energy when the forceps are operated in their coagulating mode, thus markedly improving their characteristics.

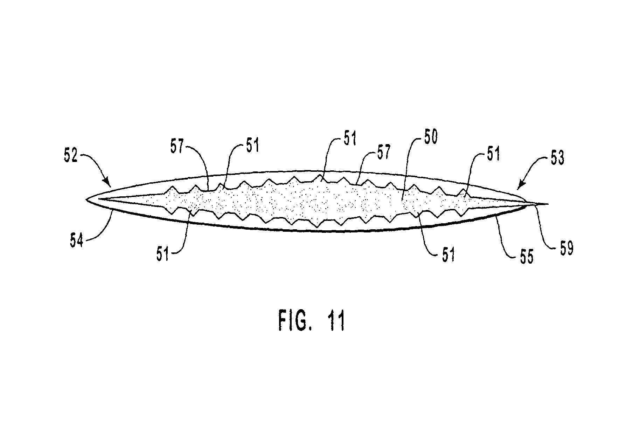

Figure 11 is a cross section illustrating a further variation on the structure of Figures 6, 9 and 10. In this embodiment, one extremity of a blade is coated, the opposite extremity includes an exposed Operative Surface as defined herein. The sides of the blade include a series of abrupt geometrical anomalies to provide relatively abrupt surface changes, thereby enhancing energy concentration. Depicted in Figure 11 is a main conductive body 50 having upper and lower surfaces containing miniature peaks 51. Peaks 51 are distributed substantially uniformly therealong so as to embody the energy concentrating characteristics hereinabove described. While end 52 is completely covered with insulation 57, end 53 has been modified to expose an operative surface 59. Accordingly, there has been included in a single electrosurgical implement a combination of the features described hereinabove: namely, a covered severing part (the left end of Figure 11); upper and lower lateral coagulating surfaces including energy concentrating peaks 51; and an exposed operative surface 59 at the right side of the implement.

It will now be evident that there has been described herein an implement which provides a marked improvement in performance.

## Claims

1. An electrosurgical electrode member (4') for performing operative procedures in a patient comprising: a conducting electrode having a main body (22') adapted for communicating radio frequency electrical energy to patient tissue for performing operative procedures thereupon, an insulating coating (20) covering said main body and energy concentration means including a working edge (23') of the main body, wherein a part of the working edge is partly sharpened and has a thickness of about 0.2mm or less for concentrating electrical energy transferred across said insulating coating from said main body to said patient tissue, and wherein the thickness of the insulating coating is sufficient to ensure that transmission of the radiofrequency electrical energy from the main body to the patient tissue is essentially exclusively by capacitive coupling.

2. A member (4') as claimed in claim 1, in which said insulating coating (20) entirely covers the working edge (23').

3. A member (4') as claimed in claim 1, in which said insulating coating (20) comprises a non-stick material.

4. A member (4') as claimed in claim 1, in which said insulating coating (20) consists essentially of the non-stick material.

5. A member (4') as claimed in claim 1, in which said insulating coating (20) comprises a fluorinated hydrocarbon material or diamond.

6. A member (4') as claimed in claim 1, in which said insulating coating (20) consists essentially of a fluorinated hydrocarbon material or diamond.

7. A member (4') as claimed in claim 1, wherein the main body further comprises an upper surface and a lower surface, wherein a plurality of peaks (51) extend from said upper surface and said lower surface.

8. A member (4') as claimed in claim 7, wherein the working edge (23') is partially covered by the insulating coating (20).

9. A member (4') as claimed in claim 7, wherein the plurality of peaks (51) are uniformly spaced along the upper surface and the lower surface.

## Patentansprüche

1. Elektrochirugisches Elektrodenteil (4') zum Durchführen operativer Verfahren an einem Patienten, umfassend: eine leitende Elektrode mit einem Hauptkörper (22'), welcher ausgestaltet ist, um eine elektrische Strahlungsenergie zu einem Gewebe eines Patienten zu übertragen, um darauf operative Verfahren auszuführen, eine Isolationsbeschichtung (20), welche den Hauptkörper und Energiekonzentrationsmittel einschließlich einer Arbeitskante (23') des Hauptkörpers überdeckt, wobei ein Teil der Arbeitskante teilweise scharf ist und eine Dicke von ungefähr 0,2mm oder weniger aufweist, um eine elektrische Energie, welche über die Isolationsbeschichtung von dem Hauptkörper zu dem Gewebe des Patienten übertragen wird, zu konzentrieren, und wobei die Dicke der Isolationsbeschichtung ausreicht, um sicherzustellen, dass die elektrische Strahlungsenergie von dem Hauptkörper zu dem Gewebe des Patienten im Wesentlichen ausschließlich durch eine kapazitive Kopplung übertragen wird.

2. Teil (4') nach Anspruch 1, wobei die Isolationsbeschichtung (20) die Arbeitskante (23') vollständig überdeckt.

3. Teil (4') nach Anspruch 1, wobei die Isolationsbeschichtung (20) ein nicht klebendes Material umfasst.

4. Teil (4') nach Anspruch 1, wobei die Isolationsbeschichtung (20) im Wesentlichen aus einem nicht klebenden Material besteht.

5. Teil (4') nach Anspruch 1, wobei die Isolationsbeschichtung (20) fluoriertes Kohlenwasserstoffmaterial oder Diamant umfasst.

6. Teil (4') nach Anspruch 1, wobei die Isolationsbeschichtung (20) im Wesentlichen aus fluoriertem Kohlenwasserstoffmaterial oder Diamant besteht.

7. Teil (4) nach Anspruch 1, wobei der Hauptkörper außerdem eine obere Oberfläche und eine untere Oberflächen umfasst, wobei eine Mehrzahl von Spitzen (51) sich von der oberen 0-berfläche und der unteren Oberflächen erstrecken.

8. Teil (4') nach Anspruch 7, wobei die Arbeitskante (23') teilweise durch die Isolationsbeschichtung (20) überdeckt ist.

9. Teil (4) nach Anspruch 7, wobei die Mehrzahl der Spitzen (51) gleichmäßig entlang der oberen Oberfläche und der unteren Oberfläche beabstandet sind.

## Revendications

1. Élément d'électrode électrochirurgicale (4' ) pour effectuer des procédures opératoires chez un patient, comprenant : une électrode conductrice comportant un corps principal (22' ) adapté pour communiquer de l'énergie électrique radiofréquence à un tissu de patient pour effectuer des procédures opératoires sur celui-ci, un revêtement isolant (20) recouvrant ledit corps principal et des moyens de concentration d'énergie comprenant une extrémité de travail (23') du corps principal, dans lequel une partie de l'extrémité de travail est en partie aiguisée et a une épaisseur d'environ 0,2 mm ou moins pour concentrer l'énergie électrique transférée à travers ledit revêtement isolant à partir dudit corps principal vers le tissu du patient, et dans lequel l'épaisseur du revêtement isolant est suffisante pour garantir que la transmission de l'énergie électrique radiofréquence à partir du corps principal vers le tissu du patient se fait essentiellement exclusivement par couplage capacitif.

2. Élément (4' ) selon la revendication 1, dans lequel ledit revêtement isolant (20) recouvre entièrement l'extrémité de travail (23').

3. Élément (4') selon la revendication 1, dans lequel ledit revêtement isolant (20) comprend un matériau non collant.

4. Élément (4') selon la revendication 1, dans lequel ledit revêtement isolant (20) est constitué essentiellement de matériau non collant.

5. Élément (4') selon la revendication 1, dans lequel ledit revêtement isolant (20) comprend un matériau en hydrocarbure fluoré ou du diamant.

6. Élément (4') selon la revendication 1, dans lequel ledit revêtement isolant (20) est constitué essentiellement de matériau en hydrocarbure fluoré ou de diamant.

7. Élément (4' ) selon la revendication 1, dans lequel le corps principal comprend en outre une surface supérieure et une surface inférieure, dans lesquelles une pluralité de crêtes (51) s'étend à partir de ladite surface supérieure et de ladite surface inférieure.

8. Élément (4') selon la revendication 7, dans lequel l'extrémité de travail (23') est partiellement recouverte par le revêtement isolant (20).

9. Élément (4') selon la revendication 7, dans lequel la pluralité de crêtes (51) est uniformément espacée le long de la surface supérieure et de la surface inférieure.
